(19) Europäisches Patentamt
European Patent Office
Office européen des brevets

(11) Publication number : **0 512 814 A1**

(12) **EUROPEAN PATENT APPLICATION**

(21) Application number : **92304076.0**

(51) Int. Cl.[5] : **A61K 7/48,** A61K 7/42

(22) Date of filing : **06.05.92**

(30) Priority : **07.05.91 GB 9109733**

(43) Date of publication of application :
**11.11.92 Bulletin 92/46**

(84) Designated Contracting States :
**AT BE CH DE DK ES FR GB GR IT LI NL PT SE**

(71) Applicant : **UNILEVER PLC**
**Unilever House Blackfriars P.O. Box 68**
**London EC4P 4BQ (GB)**
(84) **GB**

(71) Applicant : **UNILEVER N.V.**
**Burgemeester s'Jacobplein 1 P.O. Box 760**
**NL-3000 DK Rotterdam (NL)**
(84) **BE CH DE DK ES FR GR IT LI NL PT SE AT**

(72) Inventor : **Raman, Govindrajan**
**4-C Agraysar, HLRC Quarters, Chakala**
**Andheri East, Bombay 400 099 (IN)**
Inventor : **Natraj, Collur Visweswaria**
**3A Anusandhan, HLRC Quarters, Chakala**
**Andheri East, Bombay 400 099 (IN)**

(74) Representative : **Ford, Michael Frederick et al**
**MEWBURN ELLIS 2 Cursitor Street**
**London EC4A 1BQ (GB)**

(54) **Cosmetic composition.**

(57) Topical compositions for preventing or at least reducing the damaging effects of ultra-violet light on skin comprise 1-hydroxycholecalciferol and/or 1,25 dihydroxycholecalciferol in combination with a sunscreen material and preferably also retinol and/or a derivative thereof. The compositions may also be used for promoting repair of photodamaged skin.

EP 0 512 814 A1

The invention relates to a composition for topical application to human skin in order to promote the repair of photo-damaged skin and/or to reduce or prevent the damaging effects of ultra-violet light on skin. The invention also relates to the use of such compositions in the repair of photo-damaged skin and in the prevention of damage to skin due to exposure to ultra-violet light.

The treatment of human skin damaged due to exposure to ultra-violet light, ie. photo-damage, has been subject to much research effort in recent years, particularly with the realisation that skin cancer and other skin disorders can arise where the exposure to sunlight is excessive. This problem is even more serious with the depletion of the ozone layer which is believed to permit a higher level of ultra-violet radiation to reach the earth's surface.

Chronic exposure to sunlight results in multiple adverse effects on all structural elements of the skin. The clinical manifestation of these changes, collectively known as photoageing is lax, dry inelastic skin that is wrinkled and blotchy with a coarse, roughened texture.

Skin blotchiness or mottling (hyperpigmentation) which accompanies photo-ageing results from changes in the melanocytes within the population of epidermal cells. These pigment producing cells, which unlike the keratinocytes remain at the base of the epidermis, lose their normal regulation process with ageing and produce excess pigment. This leads to the formation of dense perinuclear clumps of melanin in slowly turning over keratinocytes within the epidermis, and areas of hyperpigmentation or 'age spots' develop.

In the therapy of such hyperpigmented skin, certain skin lightening agents such as kojic acid, hydroquinone or ascorbic acid are effective by inhibiting the formation of melanin. Vitamin A acid (retinoic acid) is beneficial in hyperpigmentation problems by normalising the melanocyte population.

Also by increasing cell turnover, Vitamin A acid prevents accumulation of pigment within the more rapidly dividing and migrating keratinocytes. Vitamin A acid also enhances the pigment reducing potential of conventional skin lightening agents.

The topical application of Vitamin A acid does however have a major drawback in that it is a skin irritant, and can accordingly damage the skin. Its recommended use for example as a prescription drug in the treatment of acne involves careful control, such that excessive doses are avoided in order to restrict the side effects which can occur with skin. By the same token, the use of Vitamin A acid in the treatment or prevention of photo-damaged skin is severely limited by these side effects.

As a result of a programme of screening substances other than retinoic acid for their ability to treat or prevent photo-damage of skin, without the aforementioned side effects, we have discovered that certain vitamin D derivatives are particularly effective in this respect.

The invention is accordingly concerned with compositions comprising certain derivatives of Vitamin D3, optionally in combination with Vitamin A, or derivatives thereof, and sunscreen materials, for topical application to human skin, and broadly to the use of these derivatives of Vitamin $D_3$ in the treatment of photo-damaged and/or hyperpigmented skin, and in slowing down the ageing process generally.

Derivatives of Vitamin $D_3$ have been employed in the past for topical application to skin. For example, JP 1249714 discloses lotions including cholecalciferol derivatives for promoting sun tanning. EP-A-129003 discloses cosmetic and dermatological compositions containing cholecalciferol derivatives and illustrates their efficacy, specifically for treatment of skin damaged by dermititus. The test results discussed therein show that the cholecalciferol derivatives reduced itching and scaling associated with that particular complaint. Addition of Vitamin A to such compositions is also suggested, but no test results are included to show performance of such compositions.

WO84/02845 also discloses ointments for wound healing which comprise a combination of Vitamins A and E and optionally also a Vitamin D derivative or Vitamin C. The ointments are tested for efficacy in the treatment of acne and superficial wounds and bruises and are reported as providing symptomatic relief in such applications.

However, it has now been found that certain derivatives of Vitamin $D_3$, particularly in combination with Vitamin A or derivatives thereof and/or sunscreen materials have surprising efficacy in promoting the repair of photodamaged skin.

Accordingly, the invention provides a composition suitable for topical application to human skin in order to prevent or at least reduce skin damage following exposure to ultra-violet light, which composition comprises:

(a) an effective amount of from 0.00001 to 20% by weight of a derivative of cholecalciferol chosen from 1-hydroxycholecalciferol, 1,25-dihydroxycholecalciferol and mixtures thereof; and

(b) a sunscreen material; and

(c) a cosmetically acceptable vehicle.

In another, broad aspect, the invention also provides the use of a derivative of cholecalciferol as an agent for promoting the repair of photodamaged skin.

The invention concerns a composition including a derivative of cholecalciferol (vitamin $D_3$), together with

a suitable cosmetically acceptable vehicle, which can prevent or at least reduce the damaging effects of ultra violet light on skin, when applied topically thereto. The composition can also optionally comprise retinol (vitamin A), or a derivative thereof, which together with the cholecalciferol derivative can further enhance the repair of photo damage to skin, and otherwise reduce skin blotchiness and mottling due to hyperpigmentation and generally effect an overall improvement in skin texture with reduction in fine wrinkling and improved skin colour. Co-formulation with a sunscreen enhances synergistically the photo-stability and activity of the cholecalciferol derivative within the formulation and also prevent further actinic damage to all epidermal cells and dermal tissue.

The derivative of cholecalciferol

The composition according to the invention comprises a derivative of cholecalciferol chosen from:

1-hydroxycholecalciferol,
1,25-dihydroxycholecalciferol, and
mixtures thereof

The preferred derivative is 1,25-dihydroxycholecalciferol.

The amount of the derivative of cholecalciferol which is present in the composition according to the invention should be at least that which is effective in retarding the ageing process of skin, either as a result of excessive exposure of ultra violet light, such as sunlight, or as a result of the natural ageing process. The effective amount will vary from subject to subject, but will generally lie within the range of from 0.00001 to 20%, preferably from 0.001 to 10% by weight of the composition.

The Cosmetically Acceptable Vehicle

The composition according to the invention also comprises a cosmetically acceptable vehicle to act as a dilutant, dispersant or carrier for other materials present in the composition, so as to facilitate their distribution when the composition is applied to the skin.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Propellants, such as propane, butane, isobutane, dimethyl ether, carbon dioxide, nitrous oxide;

Solvents, such as ethyl alcohol, isopropanol, acetone, ethylene glycol monoethyl ether, diethylene glycol monobutyl ether, diethylene glycol monoethyl ether;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the emulsion, and can, in the absence of other cosmetic adjuncts, form the balance of the composition.

Retinol and derivatives thereof

The composition according to the invention most preferably comprises retinol (Vitamin A) and/or a derivative thereof, further to enhance repair of photo damage to skin following exposure to ultra-violet light.

Preferably the retinol derivative comprises a retinyl ester derived from an alphatic carboxylic acid. Preferred are saturated or unsaturated acids having up to twenty carbon atoms. Example esters include:

Retinyl acetate
Retinyl butyrate
Retinyl propionate
Retinyl octanoate

Retinyl laurate
Retinyl palmitate
Retinyl oleate, and
Retinyl linoleate.

Retinyl palmitate is particularly preferred.

The amount of retinol, or a derivative thereof, when present in the composition according to the invention is from 0.01 to 10%, and preferably 0.1 to 5%, particularly 0.4 to 3% by weight of the composition.

Organic sunscreens

The composition of the invention also comprises a sunscreen material, optionally an organic sunscreen, further to enhance synergistically the benefit of the composition in providing protection from the harmful effects of excessive exposure to sunlight.

Examples of suitable organic sunscreens, when required, include those set out in Table 1 below, and mixtures thereof.

TABLE 1

| CTFA Name | Trade Name | Supplier |
|---|---|---|
| Benzophenone-3 | UVINUL M-40 | BASF Chemical Co. |
| Benzophenone-4 | UVINUL MS-40 | BASF Chemical Co. |
| Benzophenone-8 | SPECRA-SORB UV-24 | American Cyanamide |
| DEA Methoxycinnamate | BERNEL HYDRO | Bernel Chemical |
| Ethyl dihydroxy-propyl-PABA | AMERSCREEN P | Amerchol Corp. |
| Glyceryl PABA | NIPA G.M.P.A. | Nipa Labs. |
| Homosalate | KEMESTER HMS | Hunko Chemical |
| Methyl anthranilate | SUNAROME UVA | Felton Worldwide |
| Octocrylene | UVINUL N-539 | BASF Chemical Co. |
| Octyl dimethyl PABA | AMERSCOL | Amerchol Corp. |
| Octyl methoxy-cinnamate | PARSOL MCX | Bernel Chemical |
| Octyl salicylate | SUNAROME WMO | Felton Worldwide |
| PABA | PABA | National Starch |
| 2-Phenyl-benzimidazole--5-sulphonic acid | EUSOLEX 232 | EM Industries |
| TEA salicylate | SUNAROME W | Felton Worldwide |
| 3-(4-methylbenzy-lidene)-camphor | EUSOLEX 6300 | EM Industries |
| Benzophenone-1 | UVINUL 400 | BASF Chemical Co. |
| Benzophenone-2 | UVINUL D-50 | BASF Chemical Co. |
| Benzophenone-6 | UVINUL D-49 | BASF Chemical Co. |
| Benzophenone-12 | UVINUL 408 | BASF Chemical Co. |
| 4-Isopropyl dibenzoyl methane | EUSOLEX 8020 | EM Industries |
| Butyl methoxy di-benzoyl methane | PARSOL 1789 | Givaudan Corp. |
| Etocrylene | UVINUL N-35 | BASF Chemical Co. |

Further examples include substituted 1,3 diketones such as di-4-hydroxy-3-methoxycinnamolyl methane and 1,3-propanedione, 1-(4-methoxy-5-benzofuranyl)-3-phenyl.

The composition of the invention can accordingly comprise from 0.1 to 10%, preferably from 1 to 5% by weight of an organic sunscreen material.

Inorganic sunscreen

The composition according to the invention optionally can also comprise an inorganic sunscreen such as ultrafine titanium dioxide in either of two forms, namely water-dispersible titanium dioxide and oil-dispersible titanium dioxide.

Water-dispersible titanium dioxide is ultrafine titanium dioxide, the particles of which are uncoated or which are coated with a material to impart a hydrophilic surface property to the particles. Examples of such materials include aluminium oxide and aluminium silicate.

Oil-dispersible titanium dioxide is ultrafine titanium dioxide, the particles of which exhibit a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having an average particle size of less than 100nm, preferably from 10 to 40nm and most preferably from 15 to 25nm.

By topical application to the skin of a mixture of both water-dispersible ultrafine titanium dioxide and oil-dispersible ultrafine titanium dioxide, synergistically enhanced protection of the skin against the harmful effects of both UV-A and UV-B rays is achievable.

It is believed that this unexpected benefit is due to the deposition of each type of titanium dioxide on different regions of the skin surface, water-dispersible titanium dioxide being preferentially retained by hydrophilic regions of the skin's surface, while oil-dispersible titanium dioxide is retained preferentially by hydrophobic regions of the skin's surface. The combined overall effect is that more efficient physical coverage of the skin's surface is attainable and this can be demonstrated by measurement of the Sun Protection Factor (SPF).

In order to achieve the enhanced, synergistic benefit, as herein described, the weight ratio of water-dispersible titanium dioxide to oil-dispersible titanium dioxide should be from 1:4 to 4:1, preferably from 1:2 to 2:1 and ideally about equal weight proportions.

The total amount of titanium dioxide that can optionally can be incorporated in the composition according to the invention is from 1 to 25%, preferably from 2 to 10% and ideally from 3 to 7% by weight of the composition.

Other Inorganic Sunscreens

The emulsion of the invention optionally can also comprise an inorganic sunscreen in addition to or in place of ultrafine titanium dioxide as herein defined.

Examples of other inorganic sunscreens include:

zinc oxide, having an average particle size of
  from 1 to 300nm, preferably less than 100nm,
iron oxide, having an average particle size of
  from 1 to 300nm,
  silica, such as fumed silica, having an average
    particle size of from 1 to 100nm.

It should be noted that silica, when used as an ingredient in the emulsion according to the invention can provide protection from infra-red radiation.

Tocopherol

The composition according to the invention optionally can also comprise a tocopherol (vitamin E group), as an antioxidant for retinol, or a derivative, when present in the composition, and to limit oxidative damage to skin. The vitamin E group comprises $\alpha$-tocopherol, $\beta$-tocopherol, $\gamma$-tocopherol and $\delta$-tocopherol.

The amount of a tocopherol, when present in the composition according to the invention, is from 0.0001 to 20%, preferably from 0.001 to 10% by weight of the composition.

Skin lightening agent

The composition according to the invention optionally can also comprise a skin lightening agent, for lightening the colour of skin which is or has become too dark, for example following exposure to ultra-violet light.

Examples of skin lightening agents include:

L-ascorbic acid, and derivatives thereof
Kojic acid, and derivatives thereof
Hydroquinone
Extract of placenta

Compounds having the structure (2)
Arbutin
Niacin
Niacinamide, and

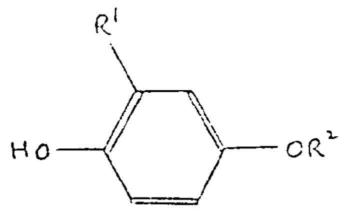

(1)

where

$R^1$ represents H, or an ether group represented by $OR^3$,

$R^2$ and $R^3$ are the same or different and each represents a group chosen from branched or unbranched alkyl or alkenyl groups having an average of from 1 to 20 carbon atoms.

The amount of skin lightening, when present in the composition according to the invention, is from 0.01 to 20%, preferably 0.1 to 10% by weight of the composition.

## OPTIONAL SKIN BENEFIT MATERIALS AND COSMETIC ADJUNCTS

A particularly convenient form of the composition according to the invention is an emulsion, in which case an oil or oily material will normally be present, together with an emulsifier to provide either a water-in-oil emulsion or an oil-in-water emulsion, depending largely on the average hydrophilic-lyophilic balance (HLB) of the emulsifier employed.

### Oil or oily material

The composition according to the invention can optionally comprise one or more oils or other materials having the properties of an oil.

Examples of suitable oils include mineral oil and vegetable oils, and oil materials, such as those already proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

### Emulsifier

The composition according to the invention can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, and the average HLB value.

Table 1

| Chemical Name of Emulsifier | Trade Name | HLB Value |
|---|---|---|
| Sorbitan trioleate | Arlacel 85 | 1.8 |
| Sorbitan tristearate | Span 65 | 2.1 |
| Glycerol monooleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Arlacel 80 | 4.3 |
| Sorbitan monostearate | Arlacel 60 | 4.7 |
| Poloxyethylene (2) stearyl ether | Brij 72 | 4.9 |
| Poloxyethylene sorbitol beeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 | 8.6 |
| PEG 400 dioleate | Tegester PEG 400-DO | 8.8 |
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Polyoxyethylene (5) sorbitan monooleate | Tween 81 | 10.0 |
| PEG 300 monooleate | Neutronyx 834 | 10.4 |
| Polyoxyethylene (20) sorbitan tristearate | Tween 65 | 10.5 |

| | | |
|---|---|---|
| Polyoxyethylene (20) sorbitan trioleate | Tween 85 | 11.0 |
| Polyoxyethylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyethylene 10 monooleate | Ethofat 0/20 | 12.2 |
| Polyoxyethylene (10) stearyl ether | Brij 76 | 12.4 |
| Polyoxyethylene (10) cetyl ether | Brij 56 | 12.9 |
| Polyoxyethylene (9.3) octyl phenol | Triton X-100 | 13.0 |
| Polyoxyethylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyethylene sorbitol lanolin derivative | G-1441 | 14.0 |
| Polyoxyethylene (12) lauryl ether | Ethosperse LA-12 | 14.4 |
| PEG 1500 dioleate | Pegosperse 1500 | 14.6 |
| Polyoxyethylene (14) laurate | Arosurf HFL-714 | 14.8 |
| Polyoxyethylene (20) sorbitan monostearate | Tween | 14.9 |
| Polyoxyethylene 20 sorbitan monooleate | Tween 80 | 15.0 |
| Polyoxyethylene (20) stearyl ether | Brij 78 | 15.3 |
| Polyoxyethylene (20) sorbitan monopalmitate | Tween 40 | 15.6 |
| Polyoxyethylene (20) cetyl ether | Brij 58 | 15.7 |

| | | |
|---|---|---|
| Polyoxyethylene (25) oxypropylene monostearate | G-2162 | 16.0 |
| Polyoxyethylene (20) sorbitol monolaurate | Tween 20 | 16.7 |
| Polyoxyethylene (23) lauryl ether | Brij 35 | 16.9 |
| Polyoxyethylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, that optionally can be incorporated in the composition of the invention is from 1 to 50%, preferably from 2 to 20% and most preferably from 2 to 10% by weight of the composition.

Water

The composition of the invention can also comprise water, usually up to 80%, preferably from 5 to 80% by volume.

Silicone Surfactant

The composition of the invention can also optionally comprise a high molecular weight silicone surfactant which can also act as an emulsifier, in place of or in addition to the optional emulsifier(s) already mentioned.

The silicone surfactant is a high molecular weight polymer of dimethyl polysiloxane with polyoxyethylene and/or polyoxypropylene side chains having a molecular weight of from 10,000 to 50,000 and having the structure:

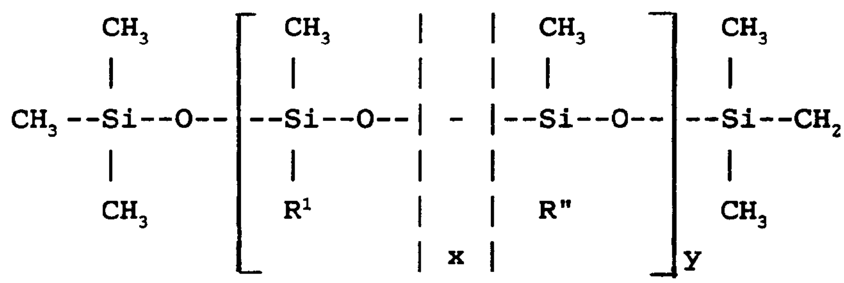

where the groups R′ and R″ are each chosen from -H, $C_{1-18}$ alkyl and

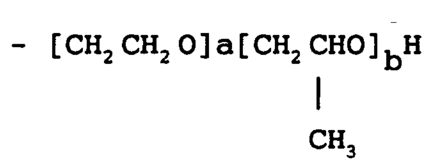

a has a value of from 9 to 115,

b has a value of from 0 to 50,
x has a value of from 133 to 673,
y has a value of from 25 to 0.25.

Preferably, the dimethyl polysiloxane polymer is one in which:
a has a value of from 10 to 114
b has a value of from 0 to 49
x has a value of from 388 to 402
y has a value of from 15 to 0.75
one of groups R′ and R″ being lauryl, and the other having a molcular weight of from 1000 to 5000.

A particularly preferred dimethyl polysiloxane polymer is one in which:
a has the value 14
b has the value 13
x has the value 249
y has the value 1.25

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the emulsion.

Other Cosmetic Adjuncts

Examples of conventional adjuncts which can optionally be employed include preservatives, such as para-hydroxy benzoate esters; antioxidants, such butyl hydroxy toluene; humectants, such as glycerol, sorbitol, 2-pyrrolidone-5-carboxylate, dibutylphthalate, gelatin, polyethylene glycol, such as PEG 200-600; buffers, such as lactic acid together with a base such as triethanolamine or sodium hydroxide; waxes, such as beeswax, ozokerite wax, paraffin wax; plant extracts, such as Aloe vera, cornflower, witch hazel, elderflower, cucumber; thickeners; activity enhancers; colourants; and perfumes. Cosmetic adjuncts can form the balance of the composition.

Use of the Composition

The composition according to the invention is intended primarily as a skin care product for topical application to human skin to repair photo-damaged skin and to prevent photo-damage to skin, which can result in premature ageing of the skin, due to exposure to sunlight. In particular, the composition can be used to reduce skin blotchiness and mottling due to hyperpigmentation, to improve skin texture with reductions in fine wrinkling and otherwise to improve skin colour and texture. In general, the composition, when topically applied to skin, is useful in the prevention of actinic damage to all epidermal cells and dermal tissue.

In use, a small quantity of the composition, for example from 1 to 5ml, is applied to exposed areas of the skin, from a suitable container or applicator and, if necessary, it is then spread over and/or rubbed into the skin using the hand or fingers or a suitable device.

PRODUCT FORM AND PACKAGING

The topical skin treatment composition of the invention can be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. The composition can be packaged in a suitable container to suit its viscosity and intended use by the consumer.

For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

The invention accordingly also provides a closed container containing a cosmetically acceptable composition as herein defined.

Evidence to Substantiate Efficacy of Substituted Cholecalciferol

Experiment 1

A series of skin creams containing a substituted cholecalciferol with or without a sunscreen and/or a retinoid were tested as follows:

0.05g of each cream was applied to an area of 16 sq.cms of depilated backs of albino guinea pigs for 3 successive days. Applications were made 15 minutes prior to irradiation and 18 hours thereafter. A Wotan sun lamp (300 W) was used at a distance of 30 cm from the skin for a period of 3 minutes. Microscopic morphology was studied on the fourth day.

Results

The results of this experiment are shown in Table 1:

TABLE 1

| | Creams | Results |
|---|---|---|
| 6. | Placebo | Thickening of the epidermis; gross scaling and sloughing |

| | | |
|---|---|---|
| 7. | Sunscreen formulation (2.5% Parsol MCX and 0.2% Parsol 1789) | Less thickening observed; cell boundaries blurred - intercellular edema. |
| 8. | 0.05% Retinol + 2.5% Parsol MCX + 0.2% Parsol 1789 | Thickened epidermis, healthy looking cells, no sloughing, compact stratum corneum. |
| 9. | 0.05% Retinoic acid + 2.5% Parsol MCX + 0.2% Parsol 1789 | Thickening epidermis, compact stratum corneum, slight scaling. |
| 10. | 0.0005% 1,25 (OH)2 cholecalciferol | Compact stratum corneum, thickened epidermis. |
| 11. | 0.0005% 1.25 (OH)2 cholecalciferol + 0.05% Retinol | Compact stratum corneum, thickened epidermis; synergy observed in extent of thickening. |
| 12. | 0.05% Retinol | Compact stratum corneum; thickened epidermis |
| 13. | 0.05% Retinoic acid | Compact stratum corneum; thickened epidermis |

Conclusions

The 1,25-dihydroxycholecalciferol and retinoids used in this experiment were effective in causing thickening of the epidermis in the presence of ultra-violet induced damage. Thickening was observed also in the absence of sunscreens. Compaction of stratum corneum is interpreted as a fortification mechanism which together with the thickened epidermis prevents further ultra-violet induced damage to the underlying dermis. Retinoic acid was itself irritant and unsuitable for cosmetic use.

Experiment 2

A further series of skin creams containing a substituted cholecalciferol, with or without a retinoid were tested as follows:

0.05g of each cream was applied to an area of 16 sq.cm on the depilated backs of albino guinea pigs. One application of cream was made each day for a total of 10 days and then microscopic morphology was carried out. The results of this experiment are shown in Table 2:

TABLE 2

| | Creams | Results |
|---|---|---|
| 1. | Placebo | No effect |
| 2. | 0.05% Retinol | Epidermal thickness increased but stratum corneum not affected. |
| 3. | 0.05% Retinoic acid | Epidermal thickness increased but stratum corneum not affected; applications had to be stopped after 5 days due to intense erythema. |
| 4. | 0.0005% 1,25-dihydroxy cholecalciferol | Epidermal thickness increased but stratum corneum not affected. |
| 5. | 0.05% Retinol + 0.0005% 1,25-dihydroxy cholecalciferol | Epidermal thickness increased but stratum corneum not affected. Synergistic effect observed in extent of thickening. |

## Conclusions

Retinol, retinoic acid and 1,25 (OH)2 cholecalciferol at the levels tested, each produced an equivalent degree of thickening of the epidermis. Retinoic acid was, however, irritant, while retinol together with the cholecalciferol derivative were synergistic in effecting thickening of the epidermis.

## EXAMPLES

The invention is further illustrated by the following examples; in each formulation, the titanium dioxide employed was ultrafine titanium dioxide having a mean particle size of from 15 to 25nm.

### Example 1

This example illustrates a lotion according to the invention.

| Ingredient | % w/w |
|---|---|
| 1,25-dihydroxycholecaliferol | 2 |
| silicone surfactant | 10 |
| volatile siloxane | 14 |
| mineral oil | 1.5 |
| titanium dioxide (water-dispersible) | 2.5 |
| titanium dioxide (oil-dispersible) | 2.5 |
| 2-hydroxy octanoic acid | 1 |
| 2-hydroxy propanoic acid | 5 |
| butylene glycol | 10 |
| sodium chloride | 2 |
| 1-proline | 0.1 |
| neutralising agent | qs |
| preservative | qs |
| perfume | qs |
| water | qs |

Example 2

This example illustrates a fluid cream according to the invention.

| Ingredient | % w/w |
|---|---|
| 1-hydroxycholecalciferol | 3 |
| volatile siloxane (DC 345) | 8.2 |
| silicone surfactant (DC 3225C) | 12 |
| petroleum jelly | 0.5 |
| mineral oil | 1.5 |
| Parsol MCX (octyl methoxycinnamate) | 3 |
| titanium dioxide (oil-dispersible) | 2 |
| titanium dioxide (water-dispersible) | 2 |
| sodium chloride | 2 |
| butylene glycol | 10 |
| l-proline | 0.1 |
| 2-hydroxy octanoic acid | 1 |
| 2-hydroxy propanoic acid | 5 |
| neutralising agent | qs |
| preservative | qs |
| perfume | qs |
| water | qs |

Example 3

This example illustrates a cream according to the invention.

| Ingredient | % w/w |
|---|---|
| 1,25-dihydroxycholecalciferol | 0.5 |
| retinol | 2 |
| volatile siloxane (DC 345 Fluid) | 8.2 |
| silicone surfactant (DC 3225C) | 12 |
| mineral oil | 1.5 |
| petroleum jelly | 0.5 |
| Parsol MCX (octyl methoxycinnamate) | 1.5 |
| titanium dioxide (oil-dispersible) | 1.0 |
| titanium dioxide (water-dispersible) | 1 |
| 2-hydroxyoctanoic acid | 1 |
| 2-hydroxypropanoic acid | 5 |
| sodium chloride | 2 |
| butylene glycol | 10 |
| 1-proline | 0.1 |
| neutralising agent (aqueous phase to 4.5) | q.s. |
| preservative | q.s. |
| perfume | q.s. |
| water | to 100 |

Example 4

This example illustrates a lotion according to the invention.

| Ingredient | % w/w |
|---|---|
| 1,25-dihydroxycholecalciferol | 1 |
| retinyl palmitate | 1 |
| Kojic acid | 1 |
| silicone surfactant (DC 3225C) | 10 |
| volatile siloxane (DC 345) | 14 |
| mineral oil | 1.5 |
| Parsol MCX | 3 |
| titanium dioxide (oil-dispersible) | 2 |
| titanium dioxide (water-dispersible) | 2 |
| butylene glycol | 10 |
| sodium chloride | 2 |
| 1-proline | 0.1 |
| 2-hydroxy octanoic acid | 1 |
| 2-hydroxy propanoic acid | 5 |
| neutralising agent | qs |
| perfume | qs |
| preservative | qs |
| water | qs |

Example 5

This example illustrates a cream in accordance with the invention.

| Ingredient | % w/w |
|---|---|
| 1,25-dihydroxycholecalciferol | 0.1 |
| Polyoxyethylene (2) stearyl alcohol | 3 |
| Polyoxyethylene (21) stearyl alcohol | 2 |
| cetyl alcohol | 1.5 |
| soft white paraffin | 1.5 |
| silicone fluid 200 | 5 |
| liquid paraffin | 8 |
| glycerin | 2 |
| preservatives | 0.5 |
| titanium dioxide (water-dispersible) | 2.5 |
| titanium dioxide (oil-dispersible) | 2.5 |
| water | to 100 |

Example 6

This example also illustrates a cream in accordance with the invention.

| Ingredients | | % w/w |
|---|---|---|
| 1,25-dihydroxycholecalciferol | | 1 |
| retinol | | 1 |
| tri-n-butyl citrate | | 2 |
| niacinamide | | 1 |
| cetyl dimethicone copolyol | ) | |
| cetyl dimethicone | ) * | 5 |
| polyglyceryl-3-oleate | ) | |
| hexyl laurate | ) | |
| isopropyl myristate | | 13.5 |
| beeswax | | 3 |
| silicone fluid 200 | | 5 |
| preservatives | | 0.5 |
| titanium dioxide (water-dispersible) | | 2.5 |
| titanium dioxide (oil-dispersible) | | 2.5 |
| water | | to 100 |

*Available is ABIL W508 ex Goldschmidt

Example 7

This example illustrates a lotion according to the invention.

| Ingredient | % w/w |
|---|---|
| 1-hydroxycholecalciferol | 0.5 |
| 1,25-dihydroxycholecalciferol | 0.5 |
| Tocopherol | 1 |
| silicone surfactant | 10 |
| volatile siloxane | 14 |
| mineral oil | 1.5 |
| ultrafine titanium dioxide (water-dispersible) | 5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

Example 8

This example illustrates a lotion according to the invention.

| Ingredient | % w/w |
|---|---|
| 1,25-dihydroxycholecalciferol | 2 |
| retinyl acetate | 0.3 |
| L-ascorbic acid | 2 |
| silicone surfactant | 10 |
| volatile siloxane | 14 |
| mineral oil | 1.5 |
| ultrafine titanium dioxide (oil-dispersible) | 5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

Example 9

This example illustrates a lotion according to the invention.

| Ingredient | % w/w |
|---|---|
| 1,25-dihydroxycholecalciferol | 1 |
| cholecalciferol | 2 |
| retinol | 2 |
| silicone surfactant | 10 |
| volatile siloxane | 14 |
| mineral oil | 1.5 |
| ultrafine titanium dioxide (water-dispersible) | 2.5 |
| ultrafine titanium dioxide (oil-dispersible) | 2.50 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

Example 10

This example illustrates an anhydrous formulation in accordance with the invention.

| Ingredient | % w/w |
|---|---|
| 1,25-dihydroxycholecalciferol | 3 |
| retinol | 2 |
| tri-n-butyl citrate | 5 |
| Kojic acid | 1 |
| Iso-propyl alcohol | 10 |
| volatile silicone | 80 |
| ethyl hexyl palmitate | 8.7 |
| antioxidant | 0.1 |

Example 11

This example illustrates a cream according to the invention.

| Ingredient | % w/w |
|---|---|
| 1,25-dihydroxycholecalciferol | 0.5 |
| retinyl palmitate | 2 |
| volatile siloxane (DC 345 Fluid) | 8.2 |
| silicone surfactant (DC 3225C) | 12 |
| mineral oil | 1.5 |
| petroleum jelly | 0.5 |
| Parsol MCX (octyl methoxycinnamate) | 1.5 |
| titanium dioxide (oil-dispersible) | 1.0 |
| titanium dioxide (water-dispersible) | 1 |
| 2-hydroxyoctanoic acid | 1 |
| 2-hydroxypropanoic acid | 5 |
| sodium chloride | 2 |
| butylene glycol | 10 |
| l-proline | 0.1 |
| neutralising agent (aqueous phase to 4.5) | q.s. |
| preservative | q.s. |
| perfume | q.s. |
| water | to 100 |

Example 12

This example illustrates a cream in accordance with the invention.

| Ingredient | % w/w |
|---|---|
| 1,25-dihydroxycholecalciferol | 0.1 |
| Retinyl linoleate | 2 |
| Polyoxyethylene (2) stearyl alcohol | 3 |
| Polyoxyethylene (21) stearyl alcohol | 2 |
| cetyl alcohol | 1.5 |
| soft white paraffin | 1.5 |
| silicone fluid 200 | 5 |
| liquid paraffin | 8 |
| glycerin | 2 |
| preservatives | 0.5 |
| titanium dioxide (water-dispersible) | 2.5 |
| titanium dioxide (oil-dispersible) | 2.5 |
| water | to 100 |

Example 13

This example illustrates an anhydrous formulation in accordance with the invention.

| Ingredient | % w/w |
| --- | --- |
| 1,25-dihydroxycholecalciferol | 3 |
| retinyl laurate | 2 |
| tri-n-butyl citrate | 5 |
| Kojic acid | 1 |
| Iso-propyl alcohol | 10 |
| volatile silicone | 80 |
| ethyl hexyl palmitate | 8.7 |
| antioxidant | 0.1 |

Example 14

This example illustrates a lotion according to the invention.

| Ingredient | % w/w |
| --- | --- |
| 1,25-dihydroxycholecalciferol | 1 |
| retinyl propionate | 2 |
| silicone surfactant | 10 |
| volatile siloxane | 14 |
| mineral oil | 1.5 |
| ultrafine titanium dioxide (water-dispersible) | 2.5 |
| ultrafine titanium dioxide (oil-dispersible) | 2.50 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

## Claims

1. A composition suitable for topical application to human skin in order to prevent or at least reduce skin damage following exposure to ultra-violet light, which composition comprises:
   (a) an effective amount of from 0.00001 to 20% by weight of a derivative of cholecalciferol chosen from 1-hydroxycholecalciferol, 1,25-dihydroxycholecalciferol and mixtures thereof; and
   (b) a sunscreen material; and
   (c) a cosmetically acceptable vehicle

2. A composition according to claim 1, which further comprises an effective amount of from 0.001% to 20% by weight of retinol or a derivative thereof.

3. A composition according to claim 1 or 2 in which the derivative of retinol is a retinyl ester.

4. A composition according to any preceding claim comprising from 0.1 to 10% by weight of an organic sunscreen material.

5. A composition according to any preceding claim comprising from 1 to 25% by weight of an inorganic sunscreen material.

6. A composition according to any preceding claim which further comprises an effective amount of from 0.00001 to 10% by weight of a tocopherol.

7. A composition according to any preceding claim, which further comprises an effective amount of a skin lightener.

8. A composition according to claim 7 comprising from 0.01 to 20% of a skin lightener.

9. Use of a derivative of cholecalciferol as defined in claim 1 and at least one of a sunscreen material, retinol and a derivative thereof in the manufacture of an agent for promoting the repair of photodamaged skin.

10. Use of a derivative of cholecalciferol as defined in claim 1 and at least one of a sunscreen material, retinol, and a derivative thereof in the manufacture of an agent for preventing or at least reducing the damaging effects of ultra-violet light on skin.

European Patent Office

# EUROPEAN SEARCH REPORT

Application Number

EP 92 30 4076

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-0 129 003 (YISSUM R&D)<br>* page 11, line 34 - page 15, line 20; claims 1,10-14; examples 5,6,13,14 *<br>--- | 1,2,6-8 | A61K7/48<br>A61K7/42 |
| D,X | WO-A-8 402 845 (ADVANCED DRUG TECHNOLOGY)<br>* page 2, line 28 - page 7, line 28; claims 1-9 *<br>--- | 1-8 | |
| X | PATENT ABSTRACTS OF JAPAN<br>vol. 12, no. 148 (C-493)(2995) 7 May 1988<br>& JP-A-62 263 110 ( SHISEIDO ) 16 November 1987<br>* abstract *<br>----- | 1,6 | |
| | | | TECHNICAL FIELDS SEARCHED (Int. Cl.5 ) |
| | | | A61K |

The present search report has been drawn up for all claims

| Place of search | Date of completion of the search | Examiner |
|---|---|---|
| THE HAGUE | 31 JULY 1992 | WILLEKENS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)